(19) **Europäisches Patentamt**

**European Patent Office**

**Office européen des brevets**

(11) **EP 0 879 249 B1**

(12) **EUROPEAN PATENT SPECIFICATION**

(45) Date of publication and mention
of the grant of the patent:
**05.09.2001 Bulletin 2001/36**

(51) Int Cl.7: **C08B 37/18**, A23L 1/00,
A61K 31/715

(21) Application number: **97902736.4**

(86) International application number:
**PCT/NL97/00047**

(22) Date of filing: **10.02.1997**

(87) International publication number:
**WO 97/29133 (14.08.1997 Gazette 1997/35)**

(54) **MODIFIED INULIN**

MODIFIZIERTES INULIN

INULINE MODIFIEE

(84) Designated Contracting States:
**BE DE FR GB IT LU NL**

(30) Priority: **09.02.1996 EP 96200299**

(43) Date of publication of application:
**25.11.1998 Bulletin 1998/48**

(73) Proprietor: **Coöperatie Cosun U.A.**
**4700 BH Roosendaal (NL)**

(72) Inventor: **KUZEE, Hendrika, Cornelia**
**NL-4388 BK Oost-Souburg (NL)**

(74) Representative: **de Bruijn, Leendert C.**
**Nederlandsch Octrooibureau**
**P.O. Box 29720**
**2502 LS Den Haag (NL)**

(56) References cited:
• **CLINICAL CHEMISTRY, vol. 13, 1967, USA,
pages 262-269, XP000573626 J. N. BEMILLER ET
AL.: "Alkaline degradation of inulin and its
structural implications"**

**EP 0 879 249 B1**

**Description**

The present invention relates to modified inulin, to a process of producing modified inulin, and to the use of modified inulin.

Native inulin, extracted from plant sources, is increasingly used in the food industry because of its attractive binding, gelling and physiological properties. Proposals have been made to use inulin derivatives in the non-food industry. Chemically modified inulins, such as oxidised inulin (WO 91/17189, WO 94/21690) (carboxyl-inulin, inulinaldehyde), carboxymethylated inulin (WO 95/15984), and hydroxyalkylated inulin (EP-A-638589), are being developed, especially for non-food applications, because of their sequestering, binding, dispersing, emulsifying and scale-inhibiting properties.

However, inulin and is modified forms have a number of drawbacks, which limit their applicability. The use of inulin in food produces discoloration upon heating, and undesired reactivity e.g. with amine compounds. Modification of inulin in alkaline medium produces an intense coloration and undesired formation of by-products. It is believed that these undesired properties are due to the presence of short-chain analogues of inulin containing reducing groups, such as glucose, fructose and fructosylfructose. As a consequence, attempts to overcome the drawbacks referred to above have focused on removing these short-chain sugars, e.g. by precipitation or chromatography. Processes of this type are disclosed in EP-A-440074 and EP-A-627490. Unwanted colour produced during modification or derivatisation of inulin can also be reduced by subsequent bleaching using activated carbon or hydrogen peroxide; however, these methods do not result in sufficient decolorisation.

It has been found that the undesired properties of inulin are not fully eliminated by the removal of short-chain reducing sugars. Inulin from which mono- and disaccharides were removed still shows discoloration upon heating and degradation in alkaline medium and undesired reactivity e.g. with amine compounds. It has been found now that these disadvantages can be overcome by treating inulin with a reducing agent. This treatment results in an inulin product having unique properties in that discolouring and degradation do not occur upon processing in food or upon further chemical or physical modification.

Inulin generally consists of a chain of β-2,1-linked anhydrofructose units (fructan) of varying chain length, terminated with a 1-linked anhydroglucose unit at the reducing end. Such chains are non-reducing because of the absence of hemiacetal groups. Native inulin contains small amounts (in the order of 10-15%) of polyanhydrofructose chains not having a glucose terminus, and thus having a reducing end. Inulin from which mono- and disaccharides were removed to below 0.3 wt.% (percentage based on monosaccharide units) were found to have a residual reducing power of 0.5 to 2.5 %. It is believed that the reduction of the relatively long-chain reducing (fructose-terminated) inulin analogues to non-reducing analogues is responsible for the surprising improvement of the properties.

Interestingly, FR-A-2707649 teaches that starch-type polysaccharides having a dextrose equivalent (DE) of less than 5, especially less than 3, can be carboxyalkylated directly, and that only if the polysaccharide has a DE of at least 5 - corresponding to a reducing power of at least 5% - a prior hydrogenation is useful.

Reduction of dahlia inulin with sodium borohydride has been described by BeMiller et al. *Clinical Chem*. **13** (1967) 261-269. They obtained an alkali-stable product containing a D-glucitol (= sorbitol) component but no mannitol component.

Hydrogenation of enzymatically obtained short-chain inulin homofructans (fructose-terminated) after separation of heterofructans (glucose-terminated) is known per se from EP-A-657106, which is concerned with producing oligofructosyl-mannitol and -sorbitol (1 to 6 fructosyl units) as low-calorie, non-cariogenic sweetener.

It has been found that reduction of inulin having a degree of polymerisation (DP) of at least 8 monosaccharide units results in a product wherein reducing groups (presumably terminal fructose units) are reduced both to glucitol (sorbitol) and mannitol units. It has furthermore been found that such reduced derivatives can be selectively removed from the inulin, resulting in the isolation of a very pure inulin of the $GF_n$ type (G = glucose, F = fructose), which is essentially free of reducing molecules of the $F_{n+1}$ type and of reduced molecules of the $RF_n$ type (R = glucitol or mannitol). Herein n+1 represents the chain length (DP). Both these new types of reduced inulin are excellent starting materials for producing further inulin derivatives on the one hand and for use as a food additive or a pharmaceutical aid on the other hand.

Thus the invention relates to an inulin modified by reduction, having an average chain length of at least 8 monosaccharide units and having a reducing power of less than 0.3 wt.% reducing groups expressed as glucose, and being derived from chicory inulin and/or having a bound mannitol content of at least 0.2 wt.%, especially at least 0.3 wt.%, and/or having a combined bound mannitol and glucitol content of less than 0.5 wt.%, especially less than 0.3 wt.%. The reducing power of the modified inulin is preferably less than 0.2 wt.%, and most preferably less than 0.1 wt. % reducing groups expressed as glucose. The modified inulin can be non-derivatised inulin, or derivatised, such as carboxymethylated inulin, as described below.

The invention also relates to a process of producing modified inulin, comprising treating inulin having an average chain length of at least 8 monosaccharide units, in particular at least 9 monosaccharide units, with a reducing

2

agent. In this description a monosaccharide unit is understood to comprise reduced groups such as glucitol and mannitol groups.

As a starting material for the process according to the invention native inulin can be used, e.g. from conventional sources such as chicory, Jerusalem artichoke, artichoke, dahlia and dandelion. Native inulin having moderate chain lengths of up to about 20 units, such as chicory inulin, is preferred. Only in exceptional cases, the average chain length may need to be increased by removal of mono-, di- and possibly trisaccharides, or by enzymic chain extension. Other prior separations, such as separation between homo- and hetero-fructans, are generally undesirable in the present process.

Reducing agents to be used in the process include conventional hydrogencontaining agents capable of reducing (hydrated) ketones or aldehydes. These include especially molecular hydrogen in the presence of a suitable catalyst, and hydride donors. Reduction can also be performed by electrochemical means. The electrochemical reduction can be performed e.g. using an amalgamated lead electrode in an alkaline or neutral medium, or, alternatively, using a graphite electrode in dilute sulphuric or other acid. The process leads to a high yield of the reduced inulin.

Catalytic hydrogenation can be performed at a pH of 4-12; pH values outside this range are undesirable because of degradation of inulin. Hydrogen can be used as such or e.g. as a nitrogen/hydrogen gas mixture. Hydrogenation is carried out in the presence of a transition metal catalyst, such as nickel, cobalt, palladium or platinum, which metals may or may not be supported. A very suitable catalyst is Raney nickel. The hydrogenation can be carried out under usual conditions, pressures ranging from 3 to 200 bar, temperatures ranging from e.g. 0°C to 100°C and reaction times ranging from about 0.5 to 48 h (depending on the other conditions such as inulin concentration, catalyst concentration, pressure and temperature) being suitable. After the hydrogenation, any dissolved catalyst is removed, e.g. by cation exchange.

Reduction with a hydride donor can be performed with conventional metal and non-metal hydrides. The preferred hydride donor is sodium borohydride, because of its commercial availability as a NaOH-stabilised aqueous solution. The reduction can be performed using e.g. 0.5-5 wt.% of sodium borohydride with respect to inulin, at a temperature of 5-90°C. The pH is preferably between 9 and 12, because borohydride is not stable at lower pH and inulin is degraded at higher pH. Reaction times will range from about 0.5 to 48 h, depending on the reaction conditions. Borates formed during the reduction can be removed, e.g. as trimethyl borates by addition of methanol, or by ion exchange.

The process according to the invention is especially suitable for the production of modified inulin derivatives, such as oxidised, carboxyalkylated, hydroxyalkylated, or cyanoethylated inulin. Prior hydrogenation of inulin results in less complicated and sometimes accelerated derivatisation reactions, less consumption of reactants, higher yields and in products having higher purity and improved properties. The derivatisation can be carried out as described before.

Oxidation of inulin per se is known e.g. from WO 91/17189, WO 94/21690 and WO 95/07303, and can be performed with hypochlorite, with periodate followed by chlorite, with hydrogen peroxide in the presence of halide; or with hypochlorite in the presence of tetramethylpiperidine-N-oxyl. Carboxymethylation of inulin is described in WO 95/15984 and can be performed with haloacetic acid or a salt thereof; carboxyethyl and carbamoylethyl groups can be introduced by reaction with acrylamide or acrylonitrile followed by hydrolysis as described in WO 96/34017. Hydroxyalkylation of inulin is described in EP-A-638589 and can be effected by reaction e.g. with ethylene oxide, propylene oxide, α-butylene oxide and higher homologues, glycidol, glycidyl esters, diepoxybutane and other epoxides. The derivatisation can result in derivatives having varying degrees of substitution, depending on the intended use. Hydroxyalkylated and carboxyalkylated inulins according to the invention preferably have 0.1-2.5 hydroxyalkyl or carboxyalkyl groups, in particular carboxymethyl groups, per monosaccharide unit. Oxidised inulins preferably have 0.2-2.6 aldehyde groups and/or, in particular, carboxyl groups per monosaccharide unit.

Pure $GF_n$ inulin can be obtained by subjecting reduced inulin to a separation step to remove components containing reduced end groups ($RF_n$). The separation can be performed by chromatography or ion exchange, e.g. using a complexing agent such as borate, tungstate or molybdate. Such pure $GF_n$ inulin is a very useful product, since it is both stable (no reducing groups) and nature-like (no chemically modified components).

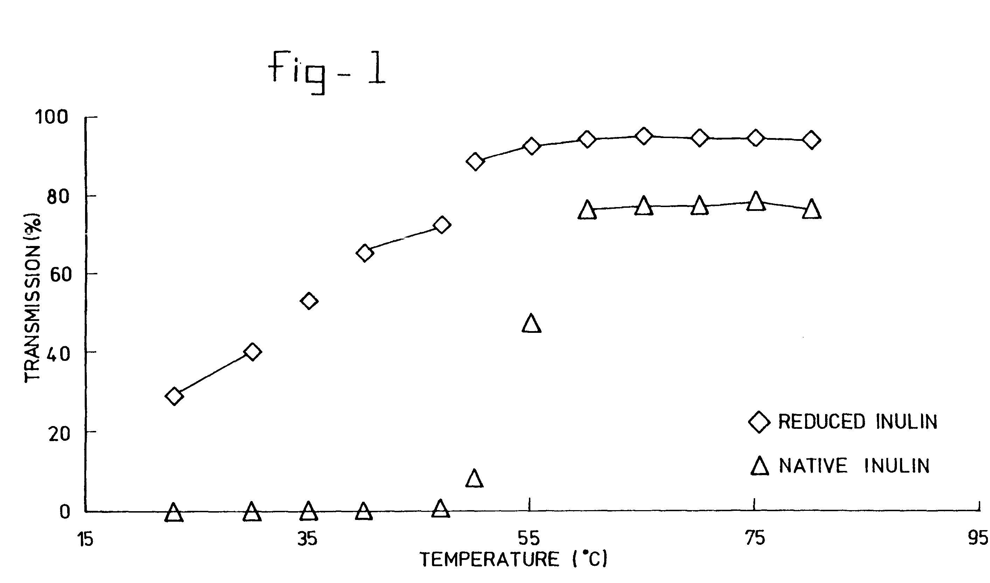

The invention also pertains to the use of the reduced inulin as a food ingredient and as a carrier for medicaments, e.g. tablets. Especially the improved taste properties (no off-taste) and improved colour properties are highly advantageous for these applications. Reduced inulin is also less cariogenic than native inulin. Thus, reduced inulin can be used as a fat replacer or as a bulk sweetener or bulking agent in sugar-free confectionery, such as chewing gum, candies, chocolate products, ice cream, fillings, and the like, as a substitute for sugar, polydextrose, as an improved fibre source in bread and health foods etc. Also, the solubility of reduced inulin is improved over native inulin. Whereas dosages of native inulin of 5% (w/w) or higher in fruit drinks and similar products result in a hazy product in a sediment within one week, reduced inulin remains clear at these levels. By way of illustration, the transmission of a 20% inulin solution/suspension in water is shown in Figure 1 (lower line: native inulin; upper line: reduced inulin). Thus, reduced inulin can be used e.g. as a fibre source in fruit drinks at higher levels than native inulin. The invention also comprises food products and pharmaceutical compositions containing reduced inulin as described above, especially at a level of at least 5 wt.% or at least 10 wt.% (medicaments).

**Examples**

*General:*

*Determination of reducing power* : The reducing power was determined using the method of Luff Schoorl. Copper (II) ions are added to a sugar matrix containing reducing sugars. The reducing sugars react with $Cu^{2+}$ to form CuO (red precipitate). Then potassium iodide and sulphuric acid are added. The iodine produced is titrated with sodium thiosulphate (starch indicator). The reducing power is read from a scale obtained with glucose standards.
*Determination of bound mannitol and glucitol content:* The product was subjected to acid hydrolysis and analysed by HPLC using an Aminex HPX 87C column.

**Example 1**

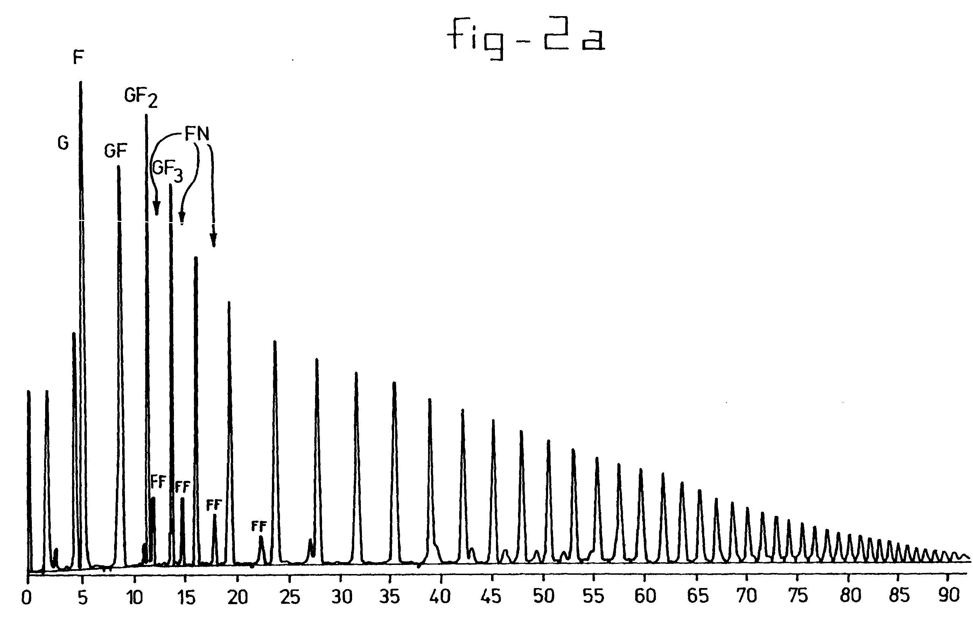

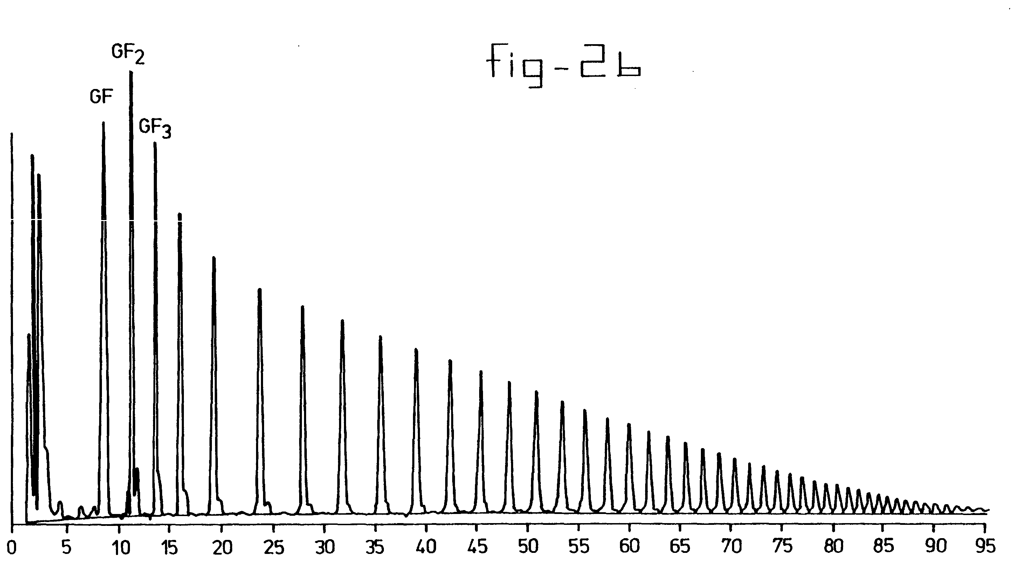

Frutafit IQ® , a commercial native inulin product (200 g), obtained from chicory, average degree of polymerisation (DP) of 10, was dissolved in 1800 ml of water. The pH was adjusted to 12.5 using NaOH. The solution was cooled to 30°C and 6.0 g of $NaBH_4$ was added. The solution was stirred overnight. The pH was then adjusted to 5.5 using 8 N HCl. The solution was desalted by electrodialysis using a P1 Aqualyzer manufactured by EIVS-Corning. The purified material was subsequently spray-dried.
The reducing power of the purified product was < 0.1% (all percentages are weight percentages unless stated otherwise). The starting native inulin had a reducing power of 7.7%. The composition of short-chain molecules of the product was as follows: sorbitol 3.1%, mannitol 2.5%, glucose 0%, fructose 0 wt.% and sucrose 4.6%. The corresponding composition of native inulin was 0%, 0,%, 1.1%, 4.6% and 4.7%, respectively. The Dionex chromatogram shows that all FF chains were reduced (see Figure 2).
The gel strength of the reduced inulin is 45 g (measured with a Stevens QTS 25), whereas the native inulin had a gel strength of 155 g. A solution of the reduced inulin at 80°C is crystal clear, whereas the same solution of native inulin is often slightly hazy.
Heating of the reduced inulin under conditions which are usual for derivatisation reactions (90°C, pH 12-13) for 2 hours resulted in a creamy white solution. In the table 1, this result is compared with other forms of inulin.

Table 1:

| Coloration of inulin in 5M NaOH at 90°C | | | |
|---|---|---|---|
| Material | Average DP [2] | Reducing power (wt.%) | Produced colour |
| Frutafit IQ® [1] | 10 | 7.7 | brown |
| Inulin after column chrom. [3] | 12 | 2.3 | red/brown |
| Inulin after ethanol prec. [4] | 21 | 1.1 | orange/red |
| Inulin after cold water prec. [5] | 30 | 0.5 | yellow/orange |
| Raftiline HP® [1] | 22 | 0.85 | orange/red |
| Reduced inulin (invention) | 10 | 0 | creamy white |

[1] Frutafit IQ and Raftiline HP are commercially available inulin products

[2] The average DP is determined using the formula:

$$\text{Av. DP} = \frac{\text{fructose after hydrolysis}}{\text{glucose after hydrolysis}} + 1$$

[3] Inulin after column chrom. = inulin from which fructose and glucose have been removed by passing an inulin solution over a cation exchange column (Bayer K1221) in sodium form

[4] Inulin after ethanol precipitation: to inulin (200 g) dissolved in water (750 ml) was added 2.5 1 of ethanol with stirring; the precipitate was collected after 16 h.

[5] Inulin after cold water precipitation: a 10 wt.% aqueous inulin solution was heated to 70°C and then cooled to 5°C; the precipitate was collected after 4 days at 5°C.

**Example 2**

*Carboxymethylation of reduced inulin*

A dry mixture of 201.7 g reduced inulin, 143.8 g sodium monochloroacetic acid and 54.3 g crushed NaOH was added to 130 ml of water with stirring. The dry substance content of the reaction is 75%. Stirring was continued for 24 h at 60°C. Then the pH was lowered to 7.0 using 6N HCl. The product was freed from NaCl, glycolic acid and diglcolic acid by means of electrodialysis. The purified material was spray-dried. No substantial amounts of byproducts were observed. The colour value of the purified material was 0.2. The colour value was determined as follows:

$$\text{colour value} = \frac{E400 - E550}{\text{concentration}} \times 100$$

(E400, E550 = extinction of the solution at 400, 550 nm;
concentration = conc. of the product in the solution in g per 100 ml) **Example 3** (comparative)

When the reaction of example 2 was carried out using native inulin, or with native inulin from which glucose and fructose had been removed, without prior reduction, the colour value of the purified material was 44.9 or 31.5, respectively. Moreover, the chromatogram of the product solution showed the presence of an unknown component (1.2 wt.%), which could not be observed when using reduced inulin.

**Example 4** (comparative)

*Bleaching of conventional carboxymethyl inulin.*

The purified product of example 3 (carboxymethylated native inulin) was decolorised using activated carbon and hydrogen peroxide as follows:
Activated carbon (4.0 g; type CN1 by Norit) was added to 100 ml of 40 wt.% aqueous carboxymethyl inulin having a pH of 7.3. After a contact period of 30 minutes at 70°C, the activated carbon was separated by filtration, and the bleaching procedure was repeated with a new batch of activated carbon.
Bleaching with hydrogen peroxide was performed by adding 2.0 and 10.0 ml, respectively, of 35% aqueous hydrogen peroxide to 100 ml of 40 wt.% aqueous carboxymethyl inulin solution having a pH of 10. After 30 minutes of bleaching at 75°C the colour was measured.
The results of the bleaching methods are summarised in table 2, together with the product of example 2 according to the invention. The results in table 2 clearly show that even strong conventional decolorization methods cannot result in a colour value obtained with the direct carboxymethyl inulin product according to the invention.

Table 2:

| Colour of carboxymethyl inulin | | |
| --- | --- | --- |
| Methods | Colour value | Elimination of colour (%) |
| Product of example 3: before bleaching | 44.9 | 0 |
| 1 x activated carbon CN1 | 9.4 | 79 |
| 2 x activated carbon CN1 | 5.4 | 88 |
| 2.0 ml 35% $H_2O_2$ | 5.8 | 87 |
| 10.0 ml 35 $H_2O_2$ | 2.2 | 95 |
| Product of example 2; without bleaching | 0.2 | 99.5*) |

*) percentage of "eliminated" (= avoided) colour with respect to product of example 3.

**Example 5**

Frutafit IQ® (2.4 kg), obtained from chicory, average degree of polymerisation (DP) of 9, was dissolved in 5.6 1 of water. The reducing power of the starting inulin was 3.7%. Raney nickel (220 g) was added as a 55% slurry. The reaction mixture was heated to 70°C and hydrogenated for 330 minutes at a pressure of 40 bar. Then the catalyst was removed by filtration. The product was subsequently spray-dried using a NIRO spray-drier. The spra-dried material had a reducing power of 0.1%. The hydrogenated product was much whiter than the starting material.

### Example 6

*Carboxymethylation of catalytically reduced inulin*

A dry mixture of 49.6 g hydrogenated inulin (example 5), 34.5 g of sodium monochloroacetic acid and 14.2 g of crushed NaOH were added to 75 ml of water with stirring. The dry substance content of the reaction was 50%. Stirring was continued for 3 h at 75°C. The pH was 11.2 after the reaction and was then lowered to 7.0 using 6N HCl. Using the Dionex AS-6 and AS-11 system, the level of lower organic acids (lactic, malic, oxalic, citric, glyceric, threonic, acetic and glyoxylic) formed was found to be 1.5 g. The product was freed from NaCl, glycolic acid and diglycolic acid by means of electrodialysis. The purified material was dried using a rotavapor. The yield of purified product was 68.1 g The degree of substitution of the purified product was 0.80. The colour value of the purified product was 0.2.

### Example 7 (comparative)

*Carboxymethylation of native inulin*

The reaction of example 6 was repeated using native inulin. During the reaction, 2.8 g of lower organic acids were formed. The pH of the reaction mixture was 10.8. After purification by electrodialysis and drying, the yield was 63.8 g. The DS of the purified product was 0.63. The colour value of the purified product was 37.4.

### Example 8

*Colour stability of carboxymethylated reduced vs. native inulin*

Carboxymethylated inulin (CMI) obtained according to the invention (example 6) (10.0 g) was dissolved in 90 ml of water in a 100 ml round-bottomed flask. The pH was adjusted to 7.0 using 0.6N HCl. The flask was placed in an oil bath at 105°C. A condenser was mounted onto the flask. The solution was refluxed for 24 h. The colour of dried CMI was measured before and after the treatment. The values were 0.3 (before) and 3.2 (after). The product was odourless.

The same procedure was followed using carboxymethylated native inulin according to the prior art (example 7). After the thermal treatment the native CMI smelled burned. The colour values were 37.4 before treatment and 71.8 after treatment.

### Example 9

*Oxidation of reduced inulin*

Frutafit IQ® (200 g) having a reducing power of 4.0% was reduced with $NaBH_4$ according to the procedure of example 1. After purification and spray-drying, 45.0 g of the reduced inulin was dissolved in 150 ml of water containing 0.02 M NaBr. 234.1 g of sodium hypochlorite solution (1.78 mmol $OCl^-$ per g) was added over a period of 2 hours. The oxidation was carried at constant temperature (20°C) and constant pH (10.5, using 5 M NaOH). After 20 h, sodium chloride was removed by electrodialysis. The purified product was dried on a rotavapor. The yield of purified material was 53.5 g. The product has a Na content of 10.0%, which corresponds to a degree of oxidation (DO) of 42%. The oxidation efficiency ($DO_{found}/DO_{theory}$) was 84%. The product had a calcium-binding power (CBP) of 0.82 mmol Ca per g of product. The CBP was determined as follows: The potential of standard solutions of $10^{-3}$ and $10^{-5}$ M $Ca^{2+}$ (containing $5.10^{-3}$ M NaCl) was determined using a calcium-selective electrode. To 150 ml of the $10^{-3}$ M $Ca^{2+}$ solution an amount of product was added which was sufficient to lower the calcium concentration to $10^{-5}$ M.
CBP (mmol) = $\{1000.(10^{-3}-10^{-5})\}/(1000.x/150)$, wherein x is amount of product used.

### Example 10 (comparative)

*Oxidation of native inulin*

The reaction of example 9 was carried out with native inulin instead of reduced inulin. The yield after purification was 52.7 g product having a Na content of 9.2% corresponding to a degree of oxidation of 39%. The oxidation efficiency was therefore 78%. The calcium binding power was 0.84 mmol Ca per g of product.

**Example 11**

*Hydroxypropylation of inulin*

A hundred grams of inulin (see below) was dissolved in 200 ml of water in a 200 ml round-bottomed flask. 12.0 g of NaOH (6 wt.% on inulin) was added to the solution. A high performance condenser and a dropping funnel were connected to the flask and the flask was placed in an oil bath at 55°C. 35.8 g of propylene oxide was added in 30 minutes with stirring. After another 20 h, the pH of the reaction mixture was reduced to 6.5 using 6N HCl. 2000 ml of acetone was added and the mixture was vigorously stirred for 30 minutes. The mixture was left standing overnight. The supernatant was decanted and the precipitate was dissolved in 150 of water. Another 2000 ml of acetone was added, the mixture was left overnight and the precipitate after decanting was dried in a vacuum oven at 70°C.

The above procedure was carried out using native inulin having a reducing power of 4.0%, using reduced inulin obtained according to the invention (example 1) having a reducing power of < 0.1%, and using reduced inulin from a different batch having a reducing power of 0.2%. Table 3 summarises the results of the three reactions.

Table 3:

| Hydroxypropylation of inulin | | | |
|---|---|---|---|
| | native | reduced | reduced |
| reducing power (%) | 4.0 | 0.2 | < 0.1 |
| yield (g) | 120.7 | 124.2 | 124.8 |
| DS | 0.57 | 0.66 | 0.68 |
| organic acids (g)[1] | 2.2 | 0.7 | 0.25 |
| colour value direct | 23.8 | 7.7 | 0.2 |
| colour value heated[2] | 50.3 | | 0.3 |

[1] amount of the following acids formed: threonic, glyceric, formic, lactic, acetic, malic and oxalic acid

[2] colour value after heating a 10% solution of pH 7.2 for 20 h at 95°C.

**Example 12**

*Pure GF$_n$ inulin*

Frutafit IQ® obtained from chicory, having a DP of 10 (300 g) was dissolved in 2700 ml of warm water. The pH was adjusted to 12.5 using NaOH. The solution was cooled to 30°C and 9.0 g of NaBH$_4$ was added. The solution was stirred overnight. After pH adjustment to 7.0 using 8N HCl, the solution was desalted by electrodialysis using a P1 Aqualyzer from EIVS-Corning.

Residual borate formed during the reduction was removed by ion exchange using the following system: a column containing 400 ml of strongly alkaline resin (MP500-OH, Bayer) followed by a column containing 200 ml of weakly acid resin (CNP80-H). The solution containing the reduced inulin was passed over the column at a rate of 800 ml/h. The column was then cluated with water. The eluate was collected and spray-dried. The material is referred to as the "cluate".

The strongly alkaline ion exchanger MP500-OH was regenerated with 500 ml 4% NaOH and washed with 500 ml of water. The pH of the collected regenerate was reduced to 7.0 and the solution was desalted by electrodialysis. The desalted solution was then spray-dried. This material is referred to as the "retentate".

The composition of the different fractions is summarized in table 4 below.

Table 4

| product | yield (g) | composition (%) | | | |
|---|---|---|---|---|---|
| | | glucose | fructose | sorbitol | mannitol |
| eluate | 255 | < 0.1 | 0.2 | 0.05 | < 0.01 |
| eluate, after acid hydrolysis | | 11.0 | 87.0 | 0.25 | < 0.01 |
| retentate | 34 | < 0.1 | < 0.1 | 5.3 | 12.9 |
| retentate after acid hydrolysis | | 0.7 | 62.3 | 17.5 | 7.9 |

Table 5

| taste | average rating | | significance |
|---|---|---|---|
| | native inulin | reduced inulin | |
| smell | 1.8 | 1.7 | |
| sweet | 2.8 | 1.9 | |
| caramel | 2.6 | 1.2 | s |
| wafer | 3.2 | 1.4 | s |
| aftertaste | 2.8 | 1.7 | s |
| body | 2.6 | 1.4 | s |

The composition of the fractions as such and after acid hydrolysis shows that inulin chains containing sorbitol or mannitol end groups are effectively retained on the strongly alkaline ion exchanger. The obtained product (eluate) is a highly purified, non-reducing, non-reduced inulin.

The eluate product (pure $GF_n$ inulin) was subjected to a taste test. A taste panel consisting of 10 persons assessed a number of taste properties of the product in a pair-wise comparison with native inulin. A rating from 1 (very little taste) to 5 (strong taste) was used. The test was carried out with solutions having a concentration of 15%. Table 5 shows the mean scores for each taste property. Reduced inulin refers to $GF_n$ inulin. An "s" indicates a significant difference.

## Example 13

*Meringues*

Meringues arc confectionery products based on an aerated structure of egg-white and sugar. A comparison was made between meringues made with sugar only, and with partial replacement by native inulin or with reduced inulin. The ingredients mentioned in table 6 were used as follows:

- blend the egg-white with 2/3 of the sugar and aerate for 3 minutes at 40°C;
- blend the inulin with remaining sugar and add this to the egg-white mass;
- shape the meringues
- bake at 140°C for 50 minutes.

The results are mentioned in the table. Partial replacement of sugar by native inulin caused an unacceptable brown colour. Meringues containing reduced inulin obtained according to the present examples had the same white colour as the reference product 1 with sugar.

Table 6

| *recipes* | *reference 1* | *reference 2* | *invention* |
|---|---|---|---|
| egg white | 30 | 30 | 30 |
| sugar | 70 | 63 | 63 |
| native inulin | - | 7 | - |
| reduced inulin | - | - | 7 |
| *results* | | | |
| firmness | + | ++ | ++ |
| colour | white | brown | white |

## Example 14

*Coffee whitener*

Evaporated milk is used as a coffee whitener. In this example, the evaporated milk was partially replaced by native inulin and reduced inulin according to table 7:

8

- dissolve the inulin in the evaporated milk
- fill in glass bottles
- sterilise at 120°C during 20 minutes

The results are mentioned in table 7. Native inulin as a fat replacer in evaporated milk showed a brown colour after sterilization. Evaporated milk with reduced inulin as the fat replacer has a normal creamy white colour. The product has an improved mouthfeel.

Table 7

| recipe | reference 1 | reference 2 | invention |
|---|---|---|---|
| evaporated milk | 100 | 95 | 95 |
| native inulin | - | 5 | - |
| reduced inulin | - | - | 5 |
| results | | | |
| taste | very creamy | creamy | creamy |
| colour | white | brown | white |

**Example 15**

Inulin can be added to white bread up to a fibre content corresponding to the fibre content of brown bread. This results in bread having the taste of white bread and being as healthy as whole-wheat bread. However, bread containing inulin is darker than normal bread, unless the oven temperature is adapted.

Bread was made using the ingredients mentioned in table 8 (weight % with respect to flour). Inulin was premixed with the flour. All ingredients were then kneaded to a smooth dough. The dough was weighed and rounded. Rising: 35 minutes at 32°C and 80% relative humidity (ERH). Punch and rise for a second period of 35 minutes. The bread was moulded and put into bread pans. Rising for 80 minutes at 34°C and 80% ERH, followed by baking at 200°C for 35 minutes (with steam). The results are given in table 8.

The bread volume can be increased by adding more gluten to the dough. It is concluded that white bread can be made with inulin following the conventional procedure, without the necessity to adapt the oven temperature.

Table 8

| ingredients | reference 1 | reference 2 | invention |
|---|---|---|---|
| wheat flour | 100 | 100 | 100 |
| yeast | 2 | 2.5 | 2.5 |
| salt | 2 | 2 | 2 |
| bread improver | 3 | 5 | 5 |
| water | 57 | 57 | 57 |
| inulin | - | 8 | - |
| reduced inulin | - | - | 8 |
| results | | | |
| volume (ml) | 3700 | 3550 | 3555 |
| colour | slightly brown | dark brown | light brown |

**Example 16**

*Tablets for pharmaceutical use*

High quality tablets can be made from 99% inulin and 1% lubricant. A drawback of high inulin levels is the off-taste of inulin. Reduced inulin according to the invention overcomes this problem.

Tablets were produced consisting of 99% inulin and 1% magnesium stearate. The inulin was either commercial inulin (Frutafit IQ®) or reduced inulin according to the present examples. The tablets were produced by intimately mixing the inulin and lubricant, tabletting the mixture in a Fette tabletting machine Exacta 1 (tablet diameter 16 mm, maximum filling depth 16 mm, compressing pressure 1200 kg, speed 50 st/min).

The tablets were subjected to a taste test. A taste panel consisting of 10 persons assessed a number of taste properties of the product. A rating from 1 (very little taste) to 5 (strong taste) was used. The test was carried out with solutions having a concentration of 15%. Table 9 shows the mean scores for each taste property. An "s" indicates a significant difference.

Table 9

| taste | average rating | | significance |
|---|---|---|---|
| | native inulin | reduced inulin | |
| sweet | 2.8 | 1.9 | |
| caramel | 2.5 | 1.2 | s |
| wafer | 3.1 | 1.5 | s |
| aftertaste | 2.8 | 1.6 | s |
| other[1] | 2.0 | 2.5 | |

[1] Other off-tastes included:
native: salt (5x), soap (3x)
reduced: burned (1x), plastic (2x)

It is concluded that tablets based on reduced inulin perform significantly better (better taste, less off-taste) than tablets based on conventional inulin.

## Description of the figures

Figure 1 represents the transmission of a 20 wt.% solution of native inulin (triangles) and reduced inulin (diamonds) at varying temperature.
Figure 2 represents the Dionex chromatograms of native inulin (above) and reduced inulin (below). G = glucose, F = fructose, GF = sucrose, $GF_2$ = kestose, $GF_3$ = nystose. The lower peaks in between correspond to $F_n$ oligomers.

## Claims

1.  An inulin modified by reduction, having an average chain length of at least 8 monosaccharide units and having a reducing power of less than 0.3 wt.% reducing groups expressed as glucose, and having a bound mannitol content of at least 0.2 wt.%.

2.  An inulin modified by reduction, having an average chain length of at least 8 monosaccharide units and having a reducing power of less than 0.3 wt.% reducing groups expressed as glucose, and being derived from chicory inulin.

3.  An inulin modified by reduction, having an average chain length of at least 8 monosaccharide units and having a reducing power of less than 0.3 wt.% reducing groups expressed as glucose, and having a combined bound mannitol and glucitol content of less than 0.5 wt.%.

4.  A modified inulin according to any one of claims 1-3, having a reducing power of less than 0.1 wt.% reducing groups expressed as glucose.

5.  An inulin modified by reduction, having an average chain length of at least 8 monosaccharide units and having a reducing power of less than 0.3 wt.% reducing groups expressed as glucose, which is further derivatised by chemical or enzymatic derivatisation. such as e.g. oxidation, carboxyalkylation, or hydroxyalkylation, or a combination thereof.

6.  A modified inulin according to claim 5, containing an average of 0.1-2.5 carboxymethyl groups per monosaccharide unit.

7.  A modified inulin according to claim 5, containing an average of 0.2-2.6 carboxyl groups per monosaccharide unit.

8.  A modified inulin according to claim 5, containing an average of 0.1-2.5 hydroxyalkyl groups per monosaccharide unit.

**9.** A process of producing a modified inulin according to any one of claims 1-4, characterised by treating inulin having an average chain length of at least 8 monosaccharide units with hydrogen in the presence of a transition metal catalyst.

**10.** A process of producing a modified inulin according to claim 3, characterised by treating inulin having an average chain length of at least 8 monosaccharide units with a reducing agent and subsequently performing a chromatography step in the presence of a complexing agent.

**11.** Use of an inulin modified by reduction, having an average chain length of at least 8 monosaccharide units and having a reducing power of less than 0.3 wt.% reducing groups expressed as glucose, as a food ingredient or as a pharmaceutical carrier.

**12.** Foodstuff containing inulin modified by reduction, having an average chain length of at least 8 monosaccharide units and having a reducing power of less than 0.3 wt.% reducing groups expréssed as glucose.

**13.** Pharmaceutical composition containing at least 5 % by weight of inulin modified by reduction, having an average chain length of at least 8 monosaccharide units and having a reducing power of less than 0.3 wt.% reducing groups expressed as glucose, as a carrier material.


**Patentansprüche**

**1.** Inulin, modifiziert durch Reduktion, welches eine mittlere Kettenlänge von mindestens 8 Monosaccharideinheiten und ein Reduktionsvermögen von weniger als 0,3 Gew.-% reduzierender Gruppen, ausgedrückt als Glucose, und einen Gehalt an gebundenem Mannit von mindestens 0,2 Gew.-% aufweist.

**2.** Inulin, modifiziert durch Reduktion, welches eine mittlere Kettenlänge von mindestens 8 Monosaccharideinheiten und ein Reduktionsvermögen von weniger als 0,3 Gew.-% reduzierender Gruppen, ausgedrückt als Glucose, aufweist und von Zichorie-Inulin abgeleitet ist.

**3.** Inulin, modifiziert durch Reduktion, welches eine mittlere Kettenlänge von mindestens 8 Monosaccharideinheiten und ein Reduktionsvermögen von weniger als 0,3 Gew.-% reduzierender Gruppen, ausgedrückt als Glucose, und einen vereinten Gehalt an gebundenem Mannit und Glucit von weniger als 0,5 Gew.-% aufweist.

**4.** Modifiziertes Inulin nach irgendeinem der Ansprüche 1-3, welches ein Reduktionsvermögen von weniger als 0,1 Gew.-% reduzierender Gruppen, ausgedrückt als Glucose, aufweist.

**5.** Inulin, modifiziert durch Reduktion, welches eine mittlere Kettenlänge von mindestens 8 Monosaccharideinheiten und ein Reduktionsvermögen von weniger als 0,3 Gew.-% reduzierender Gruppen, ausgedrückt als Glucose, aufweist und durch chemische oder enzymatische Derivatisierung, wie z.B. Oxidation, Carboxyalkylierung oder Hydroxyalkylierung oder eine Kombination davon, weiter derivatisiert ist.

**6.** Modifiziertes Inulin nach Anspruch 5, enthaltend ein Mittel von 0,1-2,5 Carboxymethylgruppen pro Monosaccharideinheit.

**7.** Modifiziertes Inulin nach Anspruch 5, enthaltend ein Mittel von 0,2-2,6 Carboxylgruppen pro Monosaccharideinheit.

**8.** Modifiziertes Inulin nach Anspruch 5, enthaltend ein Mittel von 0,1-2,5 Hydroxyalkylgruppen pro Monosaccharideinheit.

**9.** Verfahren zur Herstellung eines modifizierten Inulins nach irgendeinem der Ansprüche 1-4, **dadurch gekennzeichnet**, daß Inulin mit einer mittleren Kettenlänge von mindestens 8 Monosaccharideinheiten mit Wasserstoff in Gegenwart eines Übergangsmetall-Katalysators behandelt wird.

**10.** Verfahren zur Herstellung eines modifizierten Inulins nach Anspruch 3, **dadurch gekennzeichnet**, daß Inulin mit einer mittleren Kettenlänge von mindestens 8 Monosaccharideinheiten mit einem Reduktionsmittel behandelt wird und anschließend ein Chromatographieschritt in Gegenwart eines Komplexbildners durchgeführt wird.

11. Verwendung eines durch Reduktion modifizierten Inulins mit einer mittleren Kettenlänge von mindestens 8 Monosaccharideinheiten und einem Reduktionsvermögen von weniger als 0,3 Gew.-% reduzierender Gruppen, ausgedrückt als Glucose, als Nahrungsmittelbestandteil oder als pharmazeutischen Träger.

12. Nahrungsmittel, enthaltend durch Reduktion modifiziertes Inulin mit einer mittleren Kettenlänge von mindestens 8 Monosaccharideinheiten und einem Reduktionsvermögen von weniger als 0,3 Gew.-% reduzierender Gruppen, ausgedrückt als Glucose.

13. Pharmazeutische Zusammensetzung, enthaltend mindestens 5 Gew.-% durch Reduktion modifiziertes Inulin mit einer mittleren Kettenlänge von mindestens 8 Monosaccharideinheiten und einem Reduktionsvermögen von weniger als 0,3 Gew.-% reduzierender Gruppen, ausgedrückt als Glucose, als Trägermaterial.

**Revendications**

1. Inuline modifiée par réduction, ayant une longueur moyenne de chaîne d'au moins 8 motifs monosaccharidiques et ayant un pouvoir réducteur inférieur à 0,3 % en poids de groupes réducteurs exprimés en glucose, et ayant une teneur en mannitol lié d'au moins 0,2 % en poids.

2. Inuline modifiée par réduction, ayant une longueur moyenne de chaîne d'au moins 8 motifs monosaccharidiques et ayant un pouvoir réducteur inférieur à 0,3 % en poids de groupes réducteurs exprimés en glucose, et qui est dérivée d'inuline de chicorée.

3. Inuline modifiée par réduction, ayant une longueur moyenne de chaîne d'au moins 8 motifs monosaccharidiques et ayant un pouvoir réducteur inférieur à 0,3 % en poids de groupes réducteurs exprimés en glucose, et ayant une teneur en mannitol et glucitol liés combinée inférieure à 0,5 % en poids.

4. Inuline modifiée selon l'une quelconque des revendications 1 à 3, ayant un pouvoir réducteur inférieur à 0,1 % en poids de groupes réducteurs exprimés en glucose.

5. Inuline modifiée par réduction, ayant une longueur moyenne de chaîne d'au moins 8 motifs monosaccharidiques et ayant un pouvoir réducteur inférieur à 0,3 % en poids de groupes réducteurs exprimés en glucose, qui est en outre modifiée par modification chimique ou enzymatique, comme par exemple par oxydation, carboxyalkylation ou hydroxyalkylation, ou une combinaison de celles-ci.

6. Inuline modifiée selon la revendication 5, contenant une moyenne de 0,1 à 2,5 groupes carboxyméthyles par motif monosaccharidique.

7. Inuline modifiée selon la revendication 5, contenant une moyenne de 0,2 à 2,6 groupes carboxyliques par motif monosaccharidique.

8. Inuline modifiée selon la revendication 5, contenant une moyenne de 0,1 à 2,5 groupes hydroxyalkyles par motif monosaccharidique.

9. Procédé de préparation d'une inuline modifiée selon l'une quelconque des revendications 1 à 4, caractérisé par le fait de traiter une inuline ayant une longueur moyenne de chaîne d'au moins 8 motifs monosaccharidiques avec de l'hydrogène en présence d'un catalyseur à métal de transition.

10. Procédé de préparation d'une inuline modifiée selon la revendication 3, caractérisé par le fait de traiter une inuline ayant une longueur moyenne de chaîne d'au moins 8 motifs monosaccharidiques avec un agent réducteur puis d'effectuer une étape de chromatographie en présence d'un agent complexant.

11. Utilisation d'une inuline modifiée par réduction, ayant une longueur moyenne de chaîne d'au moins 8 motifs monosaccharidiques et ayant un pouvoir réducteur inférieur à 0,3 % en poids de groupes réducteurs exprimés en glucose, en tant qu'ingrédient alimentaire ou en tant que support pharmaceutique.

12. Denrée alimentaire contenant une inuline modifiée par réduction, ayant une longueur moyenne de chaîne d'au moins 8 motifs monosaccharidiques et ayant un pouvoir réducteur inférieur à 0,3 % en poids de groupes réducteurs exprimés en glucose.

13. Composition pharmaceutique contenant au moins 5 % en poids d'inuline modifiée par réduction, ayant une longueur moyenne de chaîne d'au moins 8 motifs monosaccharidiques et ayant un pouvoir réducteur inférieur à 0,3 % en poids de groupes réducteurs exprimés en glucose, en tant que matériau de support.

Fig - 1

EP 0 879 249 B1

fig-2a

EP 0 879 249 B1

Fig-2b